# EUROPÄISCHE PATENTANMELDUNG

(11) **EP 2 008 653 A1**
(43) Veröffentlichungstag der Anmeldung: **31.12.2008**
(21) Anmeldenummer: 08011779.9
(22) Anmeldetag: 30.06.2008
(51) Int. Cl.: A61K 31/343, A61K 31/55, A61P 25/28

(54) **Serotonerge Verbindungen zur Behandlung neuronaler Krankheiten**

(30) Priorität: 28.06.2007 DE 102007030100; 24.10.2007 DE 102007051901
(71) Anmelder: Charité-Universitätsmedizin Berlin, 10117 Berlin (DE)
(72) Erfinder: Endres, Matthias, 10115 Berlin (DE); Hörtnagl, Heide, 10717 Berlin (DE); Kronenberg, Golo, 13595 Berlin-Spandau (DE); Gertz, Karen, 13357 Berlin (DE); Balkaya, Mustafa, 10115 Berlin (DE)
(74) Vertreter: Von Renesse, Dorothea

(57) **Zusammenfassung**

Die Erfindung betrifft die Verwendung von serotonergen Verbindungen zur Behandlung neuronaler, insbesondere neurodegenerativer oder cerebrovaskulärer Erkrankungen.

## Beschreibung

Die Erfindung betrifft die Bereitstellung eines Arzneimittels zur Behandlung neuronaler Erkrankungen, insbesondere des Schlaganfalls.

Schlaganfall ist eine Volkskrankheit mit hoher Morbidität und Letalität. Allein in Deutschland treten jährlich 200.000 Neuerkrankungen auf, was den Schlaganfall zur häufigsten Ursache für Behinderungen und zur dritthäufigsten Todesursache in modernen Zivilgesellschaften macht. Die Gesamtkosten der Erkrankung werden allein in Deutschland auf etwa 10 Milliarden Euro geschätzt.

Ursache des Schlaganfalls ist zu etwa 90% ein sog. ischämischer Hirninfarkt, der durch einen akuten Gefäßverschluss im Gehirn verursacht wird. Zur kausalen Behandlung des Schlaganfalls ist derzeit lediglich die Thrombolyse mit rekombinantem Gewebe Plasminogen Aktivator (rt-PA) zugelassen. Diese Therapie ist jedoch auf ein therapeutisches Fenster von drei Stunden nach Beginn des Einsetzens der Schlaganfallsymptome beschränkt. Deutschlandweit kommen daher nur weit weniger als 5% aller Schlaganfallpatienten in den Genuss dieser Behandlung. Andere kausale Behandlungsmöglichkeiten, insbesondere Substanzen, die entweder akut oder chronisch gegeben werden, um die neurologischen Schäden zu verringern, sind derzeit trotz umfangreicher jahrzehntelanger Forschungsaktivitäten bislang in der Klinik nicht verfügbar.

Als Folge eines ischämischen Ereignisses kommt es zu einem Zelluntergang von Neuronen und anderen Zelltypen im _Gehirn.. Dies gilt selbst dann, wenn es sich nur um eine transiente Ischämie handelt und/oder die vollständige Erholung der lokalen Blutversorgung einen normalen Energiestoffwechsel und eine normale neuronale Aktivität wieder erlauben würde. Die zum Zelltod führenden pathologischen Veränderungen von Nervenzellen, die insbesondere in vulnerablen Regionen auftreten, wie z.B. dem Hippocampus, Basalganglien, oder der Großhirnrinde, manifestieren sich teilweise erst einige Tage nach der Ischämie und werden deshalb auch verzögerter neuronaler Zelltod genannt. Der neuronale Zelltod kann auch Hirnregionen betreffen, in denen der Blutstrom nicht vollständig unterbrochen war.

Die Wissenschaft und Forschung setzt bei der Behandlung von Schlaganfall daher einerseits an einer möglichst schnellen und effektiven Thrombolyse zur Wiederherstellung der Blutzufuhr, anderseits aber auch an der Verhinderung des neuronalen Zelltods an. Als Begriff für die Verhinderung des neuronalen Zelltods hat sich der Terminus "Neuroprotektion", durchgesetzt. Die Neuroprotektion betrifft somit in erster Linie die Behandlung oder Vermeidung von Sekundärerscheinungen eines ischämischen Ereignisses, die sich dem Ereignis - vor allem im Laufe der darauf folgenden Stunden und Tage - anschließen.

Der neuronale Zelltod kann nach neuerem Verständnis durch eine komplexe Kaskade einer Vielzahl einzelner Reaktionsschritte ausgelöst werden (u.a. Exzitotoxizität, Inflammation, _Apoptose sowie weitere Schadensmechanismen). Die initiale Exzitotoxizität wird dadurch ausgelöst, dass die unter Sauerstoffmangel leidenden ischämischen Neuronen schnell ATP verlieren und depolarisieren. Dies führt zu einer verstärkten postsynaptischen Abgabe des Neurotransmitters Glutamat, der seinerseits membrangebundene Glutamat-Rezeptoren aktiviert, die ihrerseits Kationen-Kanäle regulieren. Als Folge der überhöhten Glutamatausschüttung werden die Glutamat-Rezeptoren jedoch überaktiviert. Die von den Glutamat-Rezeptoren regulierten spannungsabhängigen Kationen-Kanäle werden durch die Bindung von Glutamat an den Rezeptor geöffnet. Damit setzt ein Na⁺ und ein Ca²⁺ Strom in die Zelle ein, der zu einer massiven Störung des Ca²⁺ abhängigen zellulären Stoffwechsels - einschließlich des Energiestoffwechsels - führt. Für den nachfolgend eintretenden Zelltod könnte dabei insbesondere die Aktivierung Ca²⁺ abhängiger kataboler Enzyme verantwortlich sein.

Selbst wenn der Mechanismus dieser durch Glutamat vermittelten Neurotoxizität derzeit noch nicht im Detail abschließend verstanden ist, scheint dennoch in der Fachwelt Einigkeit darüber zu bestehen, dass diese Phänomen in einem erheblichen Umfang zu dem frühen neuronalen Zelltod nach einer Ischämie beiträgt.

In den letzten Jahrzehnten wurde eine Vielzahl verschiedener Strategien entwickelt, um dem neuronalen Zelltod klinisch zu begegnen. Frühe Ansätze stellten auf die Unterdrückung des neuronalen Metabolismus und die Blockade des Calciumeinstroms ab (siehe beispielsweise EP 0 781 766 B1). Ein Großteil neuerer Strategien setzt dagegen an der Verwendung von Inhibitoren exzitatorischer Aminosäuren an. Dieser Ansatz basiert auf der Vorstellung, den ischämischen Zelltod durch die Hemmung der oben geschilderten Überanregung zu verhindern. Als Inhibitoren exzitatorischer Aminosäuren sind vor allem Glutämat-Antagonisten bekannt; die spezifisch die Rezeptoren dieser Aminosäure blockieren, sowie Verbindungen, die die Glutamatfreisetzung hemmen. Als Inhibitor des NMDA Glutamat-Rezeptors ist beispielsweise das MK-801 bekannt. Als Inhibitoren der Glutamatfreisetzung sind 2,4-Diamino-5-(2,3,5-trichlorphenyl)pyrimidin oder 2,4-Diamino-5-(2-chlorphenyl)pyrimidin bekannt (EP 0 459 830 A1).

In der Vergangenheit wurden insgesamt mehr als 50 mögliche neuroprotektive Substanzen in klinischen Versuchen zum akuten ischämischen Schlaganfall untersucht. Bislang hat sich jedoch keine der zunächst positiv in präklinischen Untersuchungen getesteten Substanzen in der klinischen Praxis als wirksam erwiesen. Demnach fehlt es derzeit immer noch an wirksamen Neuroprotektiva, die eine erfolgreiche Verhinderung oder zumindest Linderung des neuronalen Zelltods nach einem ischämischen Ereignis ermöglichen.

Nach der Reperfusion (Rekanalisation) der Blutgefäße und der möglichen Neuroprotektion - sowie der begleitenden Stabilisation des Allgemeinzustands des Patienten - noch während der Akutphase oder frühen postakuten Phase des Schlaganfallereignisses ist der behandelnde Arzt bestrebt, möglichst frühzeitig eine Rehabilitation des Patienten einzuleiten. Diese frühe Rehabilitationsbehandlung setzt üblicherweise einige Tage nach dem Schlaganfallereignis ein; und ist somit für die postakute oder chronische Phase der Behandlung typisch. Sie kann aber bestenfalls auch noch am selben Tag nach der Stabilisierung des Patienten beginnen. Eine klare zeitliche Differenzierung zwischen den einzelnen Behandlungsphasen ist daher nicht ohne weiteres möglich.

Die Rehabilitation ist in diesem Kontext definiert als eine Maßnahme, die das Gehirn unterstützt, sich durch Umorganisation oder eventuell auch Neubildung veränderten Bedingungen anzupassen, und so zunächst verlorene oder geschwächte Funktionen oder Strukturen wiederzuerlangen oder zu ersetzen. Auf der zellulären Ebene versteht der Fachmann darunter vor allem die sog. neuronale Plastizität, bei der es sich um die funktionale und/oder morphologische Anpassung reifer neuronaler Zellen auf veränderte interne oder externe Einflüsse handelt, insbesondere nach einem neuronalen Verlust oder einer Schädigung wie beispielsweise nach einem Schlaganfall. Die positive Wirkung einer Maßnahme auf die Rehabilitation oder Plastizität neuronaler Strukturen wird im vorliegenden Zusammenhang als "neuroregenerative" oder "neuroplastische" Wirkung oder als "Neuroregeneration" definiert.

Die derzeit gängigen Maßnahmen zur Rehabilitation umfassen beispielsweise ergotherapeutische, logopädische oder physiotherapeutische Behandlungen. Eine medikamentöse rehabilitative Therapie ist nicht etabliert. Letztlich versuchweise wurden stimulierende Medikamente wie Piracetam oder Amphetamine eingesetzt. Bereits unmittelbar nach der Akutphase setzt jedoch die Sekundärprävention zur Vermeidung weiterer vaskulärer Ereignisse (Schlaganfallung, Herzinfarkt etc ein). Dies geschieht z.B. mit Thrombozytenfunktionshemmern (z.B. Aspirin), Antikoagulantien (z.B. Warfarin), Cholesterinsynthesehemmern oder Blutdruckmedikamenten. Diese werden dem Patienten meist chronisch verabreicht, d.h. über Monate und Jahre.

Trotz des großen Bedarf an Maßnahmen zur Behandlung neuronaler Erkrankungen wie beispielsweise des Schlaganfalls sowohl in der akuten als auch in der postakuten oder chronischen Phase des Krankheits- oder Schadensverlaufs, haben die bisherigen Anstrengungen der Arzneimittelforschung noch nicht zu ausreichend befriedigenden Ergebnissen geführt. Der Bedarf an weiteren oder alternativen Medikamenten zur Behandlung neuronaler Schäden besteht somit trotz jahrzehntelangen Bemühungen der Wissenschaft und Forschung fort. Dies gilt insbesondere für den Bereich der postakuten und chronischen Behandlung des Schlaganfalls.

Es ist daher Aufgabe der vorliegenden Erfindung, ein Arzneimittel bereitzustellen, das hier Abhilfe schafft und die Möglichkeit eröffnet, die Behandlung neuronaler Erkrankungen, insbesondere des Schlaganfalls, zu verbessern.

Es hat sich nun überraschend gezeigt, dass die Behandlung geschädigten neuronalen Gewebes mit einer serotonergen Verbindung zu einer deutlichen Verbesserung oder Erholung der betroffenen Bereiche der Schädigung führt.

Nach der Erfindung ist dabei als "serotonerge Verbindung" jede Verbindung definiert, die zu einer Erhöhung der Wirkung von Serotonin im Zentralen Nervensystem führt. Bevorzugt handelt es sich dabei um Verbindungen, die die Serotonin-Konzentration im synaptischen Spalt gegenüber der normalen Ausgangslage erhöhen. In einer besonderen Ausführungsform der Erfindung können selektive Serotonin-Wiederaufnahme-Inhibitoren (SSRI) verwendet werden. Außerdem sind auch serotonerge Antidepressiva wie z.B. das Mirtazapin einsetzbar.

Bei der Definition der serotonergen Verbindungen im Sinne der Erfindung ist der der Verbindung jeweils zugrunde liegende Wirkmechanismus nicht relevant. Vielmehr kommt es auf das physiologische Ergebnis der erhöhten Serotonin-Wirkung im CNS an. Das kann demgemäß z.B. über eine Wiederaufnahmehemmung oder aber über eine erhöhte Ausschüttung von Serotonin erfolgen. Auch andere Mechanismen sind denkbar. Bevorzugt sind SRRI oder Mirtazapin.

Die erfindungsgemäß zu beobachtende Verbesserung gilt insbesondere für Schäden, die auf ein ischämisches Ereignis zurückgehen. Es ist dabei besonders überraschend, dass diese Verbesserung selbst dann eintritt, wenn die Gabe der serotonergen Verbindung außerhalb der akuten Phase des ischämischen Ereignisses, nämlich z.B. postakut oder chronisch erfolgt.

Die Erfindung betrifft somit die Behandlung neuronaler Erkrankungen mit serotonergen Verbindungen zu Zwecken der Neuroprotektion oder der Neuroregeneration. Beide Vorgänge spielen unter anderem bei der Behandlung des Schlaganfalls eine entscheidende Rolle, so dass die Erfindung insbesondere die Verwendung von serotonergen Verbindungen als Neuroprotektivum oder als Maßnahme zur Rehabilitation oder Neuroregeneration bei Schlaganfallpatienten umfaßt. Die Behandlung ist dabei auch postakut oder sogar chronisch möglich. Ebenso ist die Behandlung neurodegenerativer Zustände durch die Verabreichung der neuroregenerativ wirkenden serotonergen Verbindung Gegenstand der Erfindung. Dazu ist eine chronische Gabe der Verbindungen vorteilhaft.

Die erfindungsgemäße Verwendung von serotonergen Verbindungen, insbesondere von SSRI oder Mirtazapin, ist vor allem zur Behandlung von Patienten geeignet, die unter einer fokalen cerebralen Ischämie leiden.

Bei den bevorzugt einsetzbaren SSRI handelt es sich um eine Gruppe von Substanzen, die übereinstimmend am Serotonin-Transporter (5-HT Transporter) eine präferentielle Hemmwirkung entfalten, jedoch nicht notwendigerweise zu einer einheitlichen chemischen Klasse gehören. Mögliche weitere Wirkungen dieser Verbindungen an anderen Rezeptoren treten gegenüber der Wirkung am 5-HT Transporter deutlich in den Hintergrund. Da sie die Wiederaufnahme des Neurotransmitters Serotonin in die Präsynapse hemmen, erhöhen sie die Serotonin-Konzentration im synaptischen Spalt. Einige der SSRI hemmen jedoch auch schwach die postsynaptischen Rezeptoren. Das Maß der postsynaptischen Hemmung ist jedoch klinisch in der Regel nicht relevant.

Es ist eine Vielzahl von SSRI bekannt, die zum Teil auch bereits in der klinischen Praxis zur Behandlung von Depressionen eingesetzt werden. Dabei handelt es sich beispielsweise um das Fluvoxamin (Fevarin^{®}), Fluoxetin (N-Methyl-3-phenyl-3-[4-(trifluoromethyl)phenoxy]-propan-1-amin; Fluctin^{®))}, Paroxetin (3S-trans)-3-((1,3-Benzodioxol-5-yloxy)methyl)-4-(4-fluorophenyl)-piperidin, Paroxetin; Paxil^{®}, Seroxat^{®}, Sertralin, (1S,4S)-4-(3,4-Dichlorphenyl)- 1,2,3,4-tetrahydro- N-methyl-1-naphthylamin, Zoloft^{®}, Gladem^{®)}) Citalopram, 1-[3-(Dimethylamino)propyl]- 1-(4-fluorphenyl)- 1,3-dihydroisobenzofuran- 5-carbonitril] (Cipramil^{®}, Sepram^{®}), sowie dessen S-Enantiomer (Cipralex^{®}). Diese Substanzen sind erfindungsgemäß einsetzbar. Citalopram und Escitalopram sind besonders bevorzugt. Die Herstellung dieser Verbindungen ist dem zuständigen Fachmann geläufig.

Ebenso ist Mirtazapin erfindungsgemäß einsetzbar. Dabei handelt es sich um das Ebenso ist Mirtazapin erfindungsgemäß einsetzbar. Dabei handelt es sich um das *(RS)*-(±)-2-Methyl- 1,2,3,4,10,14b-hexahydropyrazino [2,1-a]pyrido[2,3-c][2]benzazepin. Auch können die dem Fachmann bekannten Antidepressiva Amitriptylin, Clomipramin, Desipramin, Doxepin, Imipramin, Maprotilin, Mianserin oder Nortriptylen erfindunsgemäß einsetzbar sein. Diese Verbindungen zeigen alle ein Rezeptoraffinitätsprofil, dass dem Grunde nach Mirtazapin entspricht. Das Rezeptoraffinitätsprofil von Mirtazapin ist unten angeführt.

Zu Zwecken der vorliegenden Erfindung werden unter "neuronalen Erkrankungen" bzw. "neuronalen Krankheiten" alle diejenigen Zustände zusammengefasst, die bezüglich der Funktionalität oder Morphologie neuronaler Gewebe, Organe oder Strukturen gegenüber einem gesunden Menschen aus medizinischer Sicht eine Veränderung darstellen. Die Ursachen der Schädigung können dabei beliebigen Ursprung sein, so z.B. genetisch bedingt oder aber auch durch ein Trauma (z. B. spirale oder cerebrale Traumata) ausgelöst sein. Diese Definition umfasst demnach auch neurodegenerative oder cerebrovaskuläre Erkrankungen. Vornehmlich psychiatrische Erkrankungen wie Depressionen sind von der vorliegenden Definition allerdings nicht erfaßt.

Als neurodegenerative Erkrankungen wird eine Gruppe von meist langsam fortschreitenden, erblichen oder sporadisch auftretenden Erkrankungen des Nervensystems verstanden. Hauptmerkmal ist der fortschreitende Verlust von Nervenzellen, der zu verschiedenen neurologischen Symptomen - darunter häufig zu Demenz und Bewegungsstörungen - führt. Die Erkrankungen können in unterschiedlichen Lebensaltern auftreten, verlaufen diffus oder generalisiert und rufen charakteristische histologische Schädigungsmuster hervor. Beispiele für diese Erkrankungen sind Morbus Alzheimer, Morbus Parkinson, Morbus Huntington oder die Amytrophische Lateralsklerose.

Als cerebrovaskuläre Erkrankungen werden im Kontext der vorliegenden Erfindung krankhafte Zustände bezeichnet, die auf einer Veränderung oder Schädigung der Blutgefäße des Zentralnervensystems beruhen, insbesondere der zuführenden Gefäße zum Gehirn. Der Schlaganfall gehört zu dieser Gruppe von Erkrankungen und wird in diesem Zusammenhang näher definiert als eine plötzlich oder innerhalb kurzer Zeit auftretende Erkrankung des Gehirns, die zu einem anhaltenden oder aber zumindest vorübergehenden Ausfall von Funktionen des Zentralen Nervensystems führt und durch kritische Störungen der Blutversorgung des Gehirns verursacht wird. Bei der ursächlichen Durchiblutungsstörung kann es sich um eine plötzlich eintretende Minderdurchblutung (z.B. Ischämie) oder aber auch um eine Blutung (z.B. hämorrhagischer Schlaganfall) handeln.

Im Kontext der vorliegenden Erfindung bezeichnet der Begriff "fokale Ischämie" bzw. "fokale cerebrale Ischämie" eine Schädigung des neuronalen Gewebes, die durch einen Verschluß oder eine Verengung einer zerebralen Arterie bedingt ist und somit zu einer lokal begrenzten Minderversorgung mit Sauerstoff und Nährstoffen führt.. Sie grenzt sich insoweit von der sog. globalen Ischämie oder der globalen cerebralen Ischämie ab, bei der ein ganzes Organ von der Sauerstoffzufuhr durch das Blut zumindest vorübergehend getrennt ist

Wie bereits ausgeführt, werden erfindungsgemäße serotonerge Verbindungen, beispielsweise SSRI, bereits als Antidepressiva eingesetzt. Mittlerweile ist aber darüber hinaus bekannt, dass SSRI ebenso als Ergänzung eines auf einer Psychotherapie basierenden Gesamtkonzepts, beispielsweise zur Behandlung der Zwangsstörung von Angstzuständen (soziale Phobie, Panikstörung, generalisierte Angststörung) oder von Essstörungen eingesetzt werden können. Ein möglicher weiterer Anwendungsbereich ist die Verminderung von Stottern. Ebenso liegen Erfahrungen im Bereich der therapeutischen Behandlung bei krankhaftem Übergewicht vor.

In der EP 0474 580 B1 wird die protektive Wirkung von SSRI auf Bewusstseinsstörungen oder Amnesie beansprucht, die im Zusammenhang mit Demenz oder cerebrovaskulären Störungen auftreten können. Um diese Schutzwirkung zu erfassen, wurde den Versuchstieren zunächst der SSRI Citalopram verabreicht, und anschließend durch den beidseitigen Verschluß der Carotiden der Tiere eine vorübergehende Unterbrechung der Blutversorgung im Gehirn erzeugt. Danach wurde unter anderem das durch die vorübergehende Ischämie erzeugte Läsionsvolumen im Gehirn oder das Verhalten der Tiere in einem Standard-Verhaltenstest untersucht. Aus den Ergebnissen aus diesem Tiermodell zur Simulation von durch einen Herzkreislaufstillstand erzeugten Schäden im Gehirn sowie an entsprechenden positiven klinischen Ergebnissen an Depressionskranken mit begleitenden Demenzerscheinungen wird die oben genannte Schutzwirkung der SSRI im Hinblick auf Bewusstseinsstörungen abgeleitet.

Darüber hinaus sind verschiedene Kombinationstherapien bekannt, in denen SSRI gemeinsam mit anderen therapeutisch aktiven Substanzen eingesetzt werden, so z.B. mit dem für die Epilepsie Behandlung zugelassenem Zonisamid zur Behandlung von Hörstörungen (siehe US 2007/021352 A1) oder in Kombination mit einem Phosphodiesterase 5 Inhibitor zur Behandlung cardiovaskulärer Erkrankungen. Die letzt genannte Kombinationstherapie ist u.a. Gegenstand der US Patentanmeldung US2006/0106039 A1. Allerdings offenbart diese Druckschrift weder die Behandlung des Schlaganfalls mit SSRI als Monotherapie, noch die nun erfindungsgemäß aufgefundene neuroprotektive oder neuroregenerative Wirkung von SSRI.

Keine dieser Druckschriften gibt demnach aus der Sicht des Fachmanns einen überzeugenden Hinweis darauf, dass - wie es Gegendstand der vorliegenden Erfindung ist - serötonerge Verbindungen, u.a. SSRI, klinisch zur Neuroprotektion nach einem fokalen Schlaganfall oder aber zur Neuroregeneration bei neuronalen Krankheiten erfolgreich einsetzbar sind.

In einer besonders vorteilhaften Ausführungsform der vorliegenden Erfindung werden die serotonergen Verbindungen nach Ablauf der akuten Phase des ischämischen Ereignisses, also in der postakuten bzw. chronischen Phase der Erkrankung verabreicht. Erfolgt die Gabe der Verbindung insbesondere auch zu Zwecken der Neurogeneration kann sie - wie bereits oben ausgeführt - auch chronisch erfolgen. Dies gilt beispielsweise bei der erfindungsgemäßen Behandlung des Morbus Alzheimer oder des Morbus Parkinson.

Dabei wird vorliegend unter der Begrifflichkeit "außerhalb der akuten" Phase derjenige Zeitraum verstanden, der sich dem Erhalt der wesentlichen Lebensfunktionen und der Stabilisierung eines Patienten nach dem ischämischen Ereignis anschließt. Diese Phase ist vor allem dadurch charakterisiert, dass sich der behandelnde Arzt der Behandlung der Folgen des ischämischen Ereignisses und der weitgehenden Regeneration der geschädigten Funktionen und Strukturen widmet. Außerhalb der akuten Phase liegt die postakute sowie die chronische Phase. Die "postakute" Phase beginnt im Sinne der vorliegenden Erfindung dann, wenn die primären Schadensereignisse und Schadenskaskaden im betroffenen Gewebe abgeschlossen sind. In der Regel ist davon auszugehen, dass die akute Phase frühestens nach etwa nach 6 bis 12 Stunden abgeschlossen ist und dann die postakute Phase beginnt.

In einer bevorzugten Ausführungsform der Erfindung erfolgt die Gabe der serotonergen Verbindung demnach frühestens 6, 12 oder 24 h nach Beginn des ischämischen Ereignisses, oder aber erst sogar einige Tagen nach Beginn des Ereignisses. Dies gilt insbesondere für die Behandlung des Schlaganfalls. Sie konnten die Erfinder zeigen, dass sogar bei einer Gabe eines SSRI beginnend am 7. Tag nach dem Schlaganfallereignis eine signifikante Verbesserung erreicht werden konnte.

Die serotonergen Verbindungen können erfindungsgemäß auch chronisch verabreicht werden. Dabei bedeutet die chronische Gabe, dass die Verbindung über einen Zeitraum von einigen Tagen bis hin zu Wochen und Monaten verabreicht werden. Besonders vorteilhaft ist die tägliche Verabreichung der Verbindung über einen kontinuierlichen Zeitraum von mehreren Tagen oder Wochen, beispielsweise über 2 Tage bis 60 Tage, vorteilhafterweise 2 bis 30 oder 5 bis 20 Tage. Sie kann jedoch auch über einen Zeitraum von mehreren Monaten bis Jahren verabreicht werden.

### Beispiele

### 1. Citalopram

Die erfindungsgemäßen neuroprotektiven und neuroregenerativen/neuroplastischen Effekte von SSRI auf neuronale Krankheiten wurde anhand eines anerkannten Tiermodells zur fokalen Ischämie in Mäusen untersucht. Dazu wurde der SSRI Citalopram den Versuchstieren in einer täglichen Dosis von 13 mg/kg Körpergewicht kontinuierlich bis zum Versuchsende verabreicht, wobei die Verabreichung am 7. Tag nach der Induktion der Ischämie einsetzte.

Anschließend wurden die SRRI Effekte auf das Volumen der ischämischen Läsionen ausgewertet. Ferner wurden die Tiere Standard-Verhaltenstests unterzogen. Zudem wurden die Konzentrationen ausgewählter Neurotransmitter erfaßt.

### Material und Methoden

### Tierversuch

### Ischämiemodell

Die Tierversuche wurden nach den Maßgaben des Tierschutzgesetzes in Übereinstimmung mit institutionellen und internationalen Richtlinien durchgeführt. Männliche 12936/SvEv-Mäuse (18-20 g, Bundesamt für Gesundheitsschutz Berlin) wurden in Gruppen von 4-5 Mäusen pro Käfig in Standardlaborkäfigen bei einer Umgebungstemperatur von 20-21°C bei einem 12 h-Licht-Dunkel-Rhythmus (8.00 Uhr/20.00 Uhr) mit freiem Zugang zu Futter und Wasser gehalten. Die Narkose der Mäuse erfolgte mit 1% Isofluran in 69% N₂0 und 30% 0₂ mit einem Narkoseverdampfer. Die Tiere wurden einer 30 min Fliamentokklusion der Arteria cerebri media (MCAo) unterworfen, wobei die Körpertemperatur und der regionale cerebrale Blutfluss wie beschrieben gemessen wurden (Endres et al., 1998). Im Rahmen einer "sham"-Operation (Scheinoperation) wurde das Filament lediglich für 30 Minuten in die linke oder rechte Arteria carotis interna eingebracht, jedoch nicht weiter vorgeschoben. Pro Versuchsgruppe wurden jeweils 10 Versuchstiere untersucht. Ein Versuchstier aus der IMCAo-(linksseitige MCAo) Gruppe verstarb nach der Operation.

### Untersuchung mittels Magnetresonanztomographie (MRT)

Die MRT-Untersuchungen wurden mit einem 7 T Bruker Scanner (Pharmascan 70/16 AS, Bruker Biospin, Ettlingen, Germany) mit einem 16 cm horizontalen Bohrlochmagnet und einem 9 cm abgeschirmten Gradienten (innerer Durchmesser) mit einer maximalen Feldstärke von 300 mT/m durchgeführt. Während der Bildgebung wurde die Maus unter Inhalationsnarkose auf einem Mausbett in die Mitte einer 38 mm RF-Spule gelegt. Die Respiration wurde mit Hilfe der Überwachungseinheit Model 1025 (SA Instruments, Inc.) überwacht. Das Ischämieareal wurde mit einer T2-gewichteten, fettsupprimierten 2D Turbo Spin-Echosequenz (TR 2937 ms; TE 36.5 ms, 12 x gemittelt) erfasst. Zwanzig aufeinander folgende axiale Schnitte mit einer Schnittdicke von 0.4 mm wurden so durch das Gehirn gelegt, dass Bulbus olfactorius sowie Kleinhirn nicht erfasst wurden. Das sogenannte "field of view" betrug 2 x 2 cm, die Matrix war 128 x 128, woraus sich eine Auflösung innerhalb einer Ebene von 156 µm ergab. Das Läsionsvolumen wurde mit der Auswertungssoftware Analyze 5.0. (AnalyzeDirect, Inc., Lenexa, USA) bestimmt. Dabei wurden die hyperintensen Ischämieareale auf den T2-gewichteten Bildern manuell bestimmt und dann das Läsionsvolumen automatisch berechnet.

### Neurologisches Defizit

Unmittelbar nach Induktion der Ischämie und zusätzlich 1 Tag vor Tötung wurden die Tiere entsprechend des Bederson Scores hinsichtlich des neurologischen Defizits "geratet"(Bederson et al., 1986).

### Verhaltensphänotypisierung

Ab zwölf Wochen nach MCAo/Reperfusion wurden die Tiere in Folge einer Reihe von Verhaltenstests unterworfen: spontane lokomotorische Aktivität, "elevated plus maze", modifizierter Porsolt Schwimmtest, "light/dark box", "latency to feed" sowie Konsum von Zuckerwasser. Die Verhaltenstests wurden während der Dunkelperiode (d.h. während der Aktivitätsphase der Tiere) durchgeführt. Vor Untersuchungsbeginn hatten die Tiere jeweils mindestens eine Viertelstunde Zeit, sich an den Untersuchungsraum zu gewöhnen. Die Reihenfolge der Verhaltensuntersuchungen entsprach dabei früheren Empfehlungen, diese in einer Graduierung nach dem Ausmaß der Stressbelastung mit Beginn mit den am wenigsten belastenden Tests durchzuführen (van Gaalen und Steckler, 2000; McIlwain et al., 2001). Der Untersucher war während der Durchführung der Experimente hinsichtlich der Versuchsgruppen verblindet. Die Tiere wurden 2 Tage nach dem Abschluss der Verhaltenstestung geopfert und die Gehirne für die weitere Aufarbeitung asserviert (Bestimmung von Monoaminen, Aminosäuren, Neurotrophinkonzentrationen). Separate Versuchsgruppen wurden für die Immunhistochemie, Bestimmung der cerebralen Läsionsgrößen, Immunoblots und RT-PCR angesetzt (siehe unten).

### Video Tracking

Bei allen Tests wurde ein automatisches Videotracking eingesetzt (TSE VideoMot 2 System, Technical & Scientifical Equipment GmBH, Bad Homburg, Deutschland) mit einer räumlichen Auflösung von 640 x 480 Pixel und einer zeitlichen Auflösung von 1 Hz (spontane lokomotorische Aktivität) bzw. 12.5 Hz ("elevated plus maze").

### Spontane lokomotorische Aktivität

Die Tiere wurden in Einzelkästen gesetzt (30 x 20 x 15 cm). Zeit, Geschwindigkeit und Distanz für spontane lokomotorische Aktivität wurde über eine Zeitspanne von 8 Stunden über Nacht automatisch mittels VideoMot aufgezeichnet und ausgewertet. Der Test fand unter gedimmter indirekter Beleuchtung in einer neutralen schallgeschützten Umgebung statt.

### Elevated Plus Maze

Das sogenannte "elevated plus maze" ist weithin als Test zur Bestimmung von Ängstlichkeit bei Nagern etabliert. Die Untersuchung wurde nach publizierten Protokollen durchgeführt (Lister et al., 1987; Rodgers et al., 2002). Das "Maze" ist aus schwarzem Plexiglass gefertig und besteht aus zwei sich gegenüberstehenden offenen Armen (30 x 5 x 0.25 cm) sowie zwei sich gegenüberstehenden geschlossenen Armen (30 x 5 x 15 cm). Die Arme gehen von einer gemeinsamen Plattform aus (5 x 5 cm), die auf einem Ständer 50 cm oberhalb des Bodens befestigt ist. Das Plus Maze wird für das Experiment in eine schallgeschützte und neutrale Umgebung mit indirekter Beleuchtung gebracht. Die Tiere werden zu Beginn des Test in das Zentrum des "Maze" gesetzt, wobei der Kopf in die Richtung eines geschlossenen Arms positioniert wird. Die (i) Gesamtzeit, die das Tier in den offenen Armen verbringt, sowie (ii) die Anzahl der Besuche in den offenen Armen ("open arm visits") wurden über einen Zeitraum von 5 min aufgezeichnet. Die Zeit in den offenen Armen ist als aussagekräftiger Index für Ängstlichkeit etabliert.

### "Tail Suspension Test"

Das Versuchstier wird etwa 1 cm vom Schwanzende mit Klebestreifen (3M Durapore) an einer Metallstange 80 cm über dem Boden befestigt. Die Zeitdauer, in der das Tier immobil war, wurde durch einen verblindeten Beobachter aufgezeichnet. Immobilität war dabei definiert als Abwesenheit von Bewegungen unter Nichtberücksichtigung der Atembewegungen. Wenn eine Maus an ihrem Schwanz nach oben kletterte, wurde sie sanft wieder nach unten gezogen und die Testung anschließend fortgesetzt. Mäuse, die auch weiterhin an ihrem Schwanz nach oben kletterten, wurden von der Auswertung ausgeschlossen.

### Modifizierter Porsolt Schwimmtest

Der modifizierte Porsolt Schwimmtest stellt ein Screeningverfahren für Depressivität und für die Wirksamkeit von Antidepressiva dar. Die Testung wurde im wesentlichen wie beschrieben durchgeführt (Cryan et al., 2002; Lucki et al., 1997). Die Tiere wurden einzeln für 5 min in ein gläsernes Gefäß gegeben (15 x 21 cm), das mit 22°C warmen Wasser gefüllt war (8.5 cm Tiefe). Über diesen Zeitraum von 300 sec wurden dann die Verhaltensweisen "Schwimmen", "Climbing" (Klettern) sowie "Floating" (Treiben) gemonitort. "Schwimmen" war definiert als Bewegung (gewöhnlich horizontal) durch das Gefäß. Immobilität war definiert als Bewegungslosigkeit unter Nichtberücksichtigung solcher Bewegungen, die das Tier daran hindern unterzugehen. "Climbing" (Klettern), bestand in nach oben gerichteten Bewegungen der Vorderpfoten des Tieres am Gefäßrand. Es wurden Zeit und Latenz der drei Verhaltensweisen verglichen und wie publiziert ausgewertet (Cryan et al., 2002; Bale und Vale, 2003; Montkowski et al., 1997; Lucki et al., 1997).

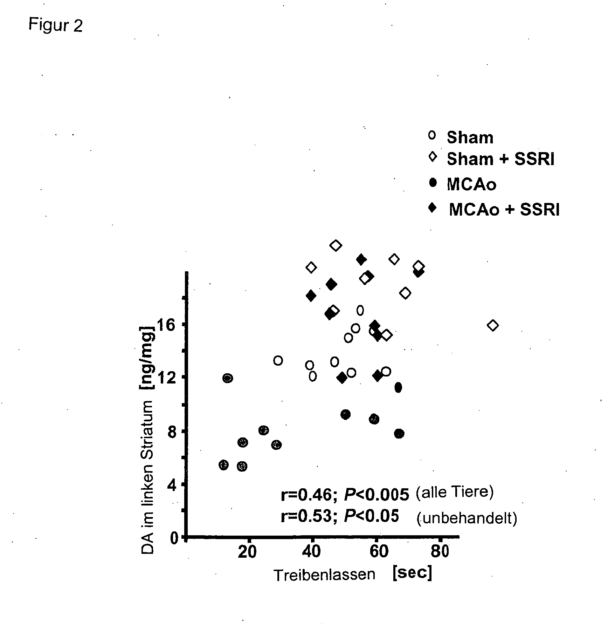

Die MCAo Tiere zeigen im sog. "Schwimmtest" (forced swim test) nach Porsolt kürzere Latenzen bis zum ersten Treibenlassen (latency to float) im Vergleich zu Sham Tieren. Dieser "depressive", Phänotyp wird durch die Behandlung mit dem SSRI Citalopram gänzlich verhindert. Darüberhinaus zeigen MCAo Tiere, nicht aber Citalopambehandelte MCAo Tiere, signifikant niedrigere Gewebsspiegel von Dopamin im ischämischen Striatum. Hierbei korreliert der Dopamingehalt mit dem Verhalten im "Forced swim test" (Pearson Correlation) (Figur 2).

### "Light-dark" Box

Die Light/Dark-Box (LD Box) bestand aus einem Plexiglassapparat (TSE shuttle box System) mit den Maßen 30 cm x 17 cm x 17 cm (Länge x Breite x Höhe) und war in zwei Kompartimente eingeteilt (abgedunkeltes bzw. voll mit einer 20W Glühbirne ausgeleuchtetes Kompartiment). Die Tiere wurden über 5 Minuten in der LD Box getestet. Eine kleine Öffnung innerhalb der Trennwand zwischen den beiden Räumen (4 x 4 cm) erlaubte den Tieren einen freien Wechsel hin und her zwischen den beiden Kompartimenten. Die Tiere wurden für einen Zeitraum von 5 Minuten in den LD Box untersucht. Die Tiere wurden zu Untersuchungsbeginn in den abgedunkelten Raum gesetzt. Danach wurden gemessen (i) Anzahl von Wechseln in das ausgeleuchtete Kompartiment, (ii) Gesamtzeit, welche die Tiere im abgedunkelten Kompartiment verbrachten, (iii) Gesamtzahl der Wechsel zwischen den beiden Kompartimenten, (iv) Lokomotion. Alle Maße wurden durch die Lichtsensoren des Verhaltensapparates erfasst und von der Software des Systems ausgewertet (System Shuttle ver.02.10)

### "Novelty-suppressed feeding test"

Die Experimente würden in einem schallgeschützten, hell ausgeleuchteten (1500 lx) Beobachtungsraum zwischen 11.00 und 12.00 Uhr in einem hölzernen weißen "open field" (120 cm x 120 cm x 40 cm) durchgeführt. Beginnend 48 Stunden vor Testung wurde den Tieren Futter vorenthalten. Bereits eine Stunde vor der Testung wurden die Tiere in den Beobachtungsraum gebracht. Eine Petrischale (Durchmesser 8 cm) wurde mit Standardmäusepellets gefüllt und in die Mitte des "open field" gestellt. Jedes Tier wurde mit Blick auf das Futter einzeln in eine Ecke des unbekannten "open field" gesetzt. Das Verhalten der Tiere wurde aufgezeichnet und hinsichtlich der Dauer bis zum Beginn der Nahrungsaufnahme ausgewertet. Tiere, die sich nicht bewegten bzw. die während des Untersuchungszeitraums von 300 s nichts fraßen wurden von der Auswertung ausgeschlossen.

### Sucrose Consumption Test (Zuckerpräferenz)

Hierbei handelt es sich auch um einen sehr einfachen, nichtinvasiven Test, bei dem nach einem etablierten Verfahren getestet wird, ob Mäuse eine 1 %ige Sucrose-Lösung Leitungswasser vorziehen (Strekalova et al., 2004). Für einen Zeitraum von 24 h wurde den Tieren innerhalb ihres gewohnten Käfigs die freie Auswahl zwischen einer Zuckerlösung und Wasser mittels zweier sonst identischer Wasserflaschen gegeben. Hierbei wurden die Flaschen jeweils vor und am Ende des Versuchsprotokolls gewogen. Hieraus lässt sich dann leicht die Sucrosezufuhr in mg/g Körpergewicht errechnen.

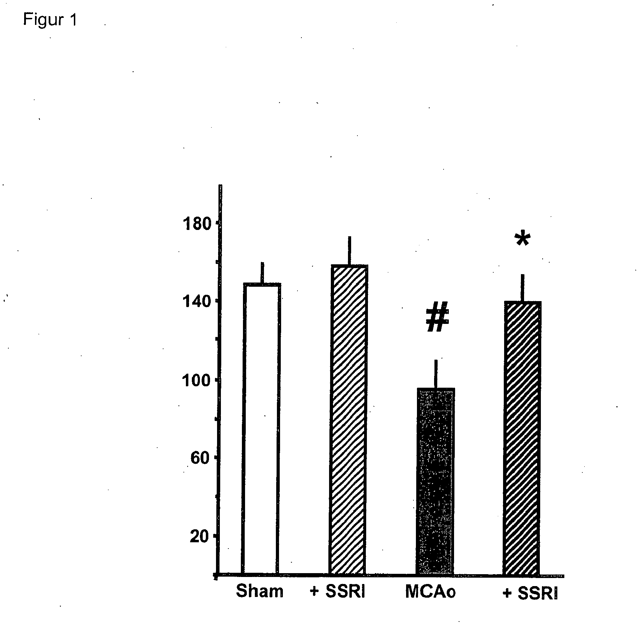

Viele Wochen nach einem 30 min Verschluß der linken A. cerebri media (MCAo) mit nachfolgender Perfusion nehmen 129/SV Wildtyp Mäuse im "Sucrose consumption test" weniger gesüßtes Wasser im Vergleich zu Schein (sham)-operierten Mäusen zu sich. Dieser anhedone Phänotyp wird durch die Behandlung mit dem SSRI Citalopram vollständig verhindert (Figur 1).

### Motorik

Die motorische Koordination wurde mit Hilfe eines sich beschleunigenden Tretrades (Rota Rod Treadmill for mice; Durchmesser der sich beschleunigenden Stange 3 cm; TSE Systems, Bad Homburg, Deutschland) einen Tag nach Durchführung der Morris-Wasserlabyrinthexperimente getestet. Nachdem die Tiere sich zunächst an einem Tag vorab bei einer konstanten Geschwindigkeit von 4 Umdrehungen pro Minute an das "rota rod" gewöhnen konnten (3 Durchgänge von jeweils 120 s mit einer zwischenzeitlichen Pause von 2 h) wurde am nächsten Tag der eigentliche Test durchgeführt. Die Mäuse wurden bei einer Anfangsgeschwindigkeit von 4 Umdrehungen pro Minute auf die rotierende Stange gesetzt. Die Höchstgeschwindigkeit von 40 Umdrehungen pro Minute wurde nach 245 s erreicht, die maximale Untersuchungszeit betrug 300s. Gemessen wurde die Zeit, bis die Maus sich nicht mehr auf der rotierenden Stange halten konnte und herunterfiel.

### Neurochemische Auswertung

### Aufarbeitung des Gewebes

Bei Versuchsende wurden die Tiere dekapitiert und die Hirne rasch entnommen. Gewebeproben aus beiden Hemisphären wurden schnell aus Cortex (fronto-parietal), Striatum (Nucl. Caudatus/ Putamen), Septum und Hippokampus (insgesamt acht Gewebeproben je Gehirn) genommen, in flüssigem Stickstoff schockgefroren und bis zur weiteren Untersuchung bei -80°C aufbewahrt. Für die neurochemischen Analysen wurden die gefrorenen Gewebeproben gewogen und mit Ultraschall in 10-20 vol deionisiertem Wasser bei 4°C homogenisiert. Für die Bestimmung von Monoaminen wurde ein Aliquot des Homogenats sofort zu demselben Volumen 0.2 N Perchlorsäure gegeben und bei 25000g für 10 min bei 4°C zentrifugiert. Der Überstand wurde für die Messung von Monoaminen und ihren Abbauprodukten verwendet. Für die Bestimmung von Aminosäuren wurden 30 µl wässrigen Homogenats zu 120 µl Methanol gegeben und für 10 Minuten bei einer Temperatur von 4°C mit 25000g zentrifugiert, um präzipitiertes Protein zu entfernen. Das zurückbleibende wässrige Homogenat wurde verwendet zur Bestimmung der Cholinacetyltransferase (ChAT)-aktivität.

### Neurochemie

Die Konzentrationen von Monoaminen (Noradrenalin, NA; Dopamin, DA; 5-Hydroxytryptamin, 5-HT und ihrer Metaboliten 3,4-Dihydroxyphenylessigsäure, DOPAC; Homovanillinsäure, HVA; 5-Hydroxyindolessigsäure, 5-HIAA) wurden entsprechend früher beschriebenen Methoden mittels HPLC mit elektrochemischer Detektion bestimmt (Felice et al., 1978; Sperk et al, 1981, 1982). Glutamat, Gammaaminobuttersäure (GABA) und Taurin wurden wie publiziert gemessen (Piepponen und Skujins, 2001). Die Aktivität der ChAT wurde entsprechend der Arbeit von Fonnum (1975) mit unwesentlichen Modifikationen bestimmt. Die Aktivität der ChAT wird angegeben als Mikroeinheiten (µU; Picomol Acetylcholinbildung pro Minute) je Milligramm Gewebe (Feuchtgewicht).

Die MCAo Tiere zeigen einen signifikant erniedrigten Gewebespiegel von Dopamin im ischämischen Striätum. Dabei korreliert der Dopamingehalt mit dem Verhalten im Schwimmtest nach Porsolt (Figur 2).

### Neurotrophinbestimmungen

Gewebeproben wurden durch Ultraschallbehandlung in 10-20 vol Lysispuffer (0.1M Tris-HCl, pH 7.0, 0.4M NaCl, 0.1% NaN3, und einer Reihe von Proteaseinhibitoren) homogenisiert. Die Konzentration von BDNF und NGF auf Proteinebene wurde mittels eines kommerziellen ELISAs bestimmt (Promega). Die Nachweisgrenze des Tests lag bei 1pg/ml bzw. 0.25pg/ml. NGF und BDNF-Konzentrationen wurden mittels eines fluoreszenzbasierten ELISA wie publiziert beschrieben (Hellweg et al., 2003).

### Immunhistochemie

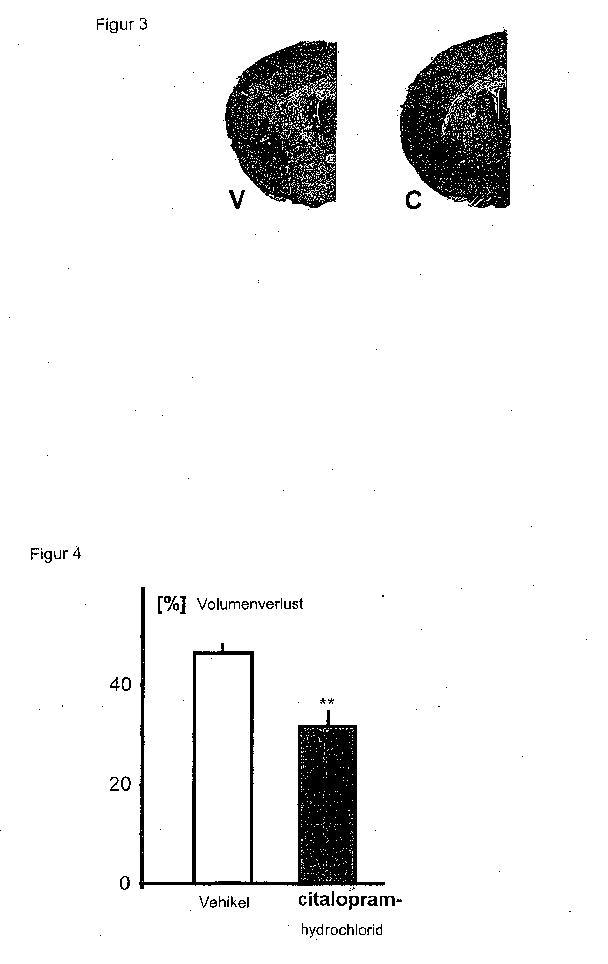

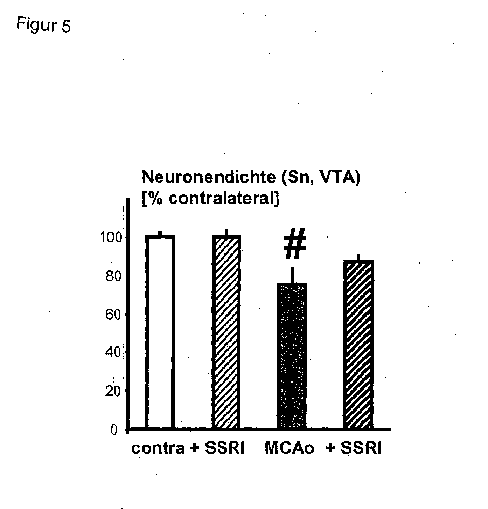

Nach tiefer Narkose wurden die Tiere transkardial mit 4% Paraformaldehyd in 0.1M Phosphatpuffer getötet. Koronare 40 µm Schnitte wurden an einem Schlittenmikrotom (Leica, Bensheim, Deutschland) gewonnen. Die Schnitte wurden frei flottierend gefärbt. Die Schnitte wurden für die lichtmikroskopische Auswertung nach der ABC-Methode (Vectastain Elite Kit, Vector Laboratories) gefärbt und mit Diaminobenzidin als Chromogen entwickelt. (Figur 3, Figur 4 und Figur 5).

129/SV Wildtypmäuse wurden einer 30 min MCAo mit nachfolgender Reperfusion ausgesetzt. Mäuse, die eine chronische Behandlung mit dem SSRI Citalopram (Dosis und Protokoll siehe Methoden) erhalten hatten, wiesen nach 3 Monaten signifikant kleinere Läsionen auf (bestimmt mittels immunhistochemischer Färbung mit dem neuronalen Marker NeuN). Weiterhin zeigten die mit Citalopram behandelten Tiere einen signifikant geringeren Verlust des Volumens (geringere Atrophie) des ipsilateralen Striatum und einen signifikant geringeren Verlust von Mittelhirnneuronen (in der Substantia nigra sowie weiteren Kerngebieten) als MCAo Mäuse, die mit der Kontrollsubstanz behandelt wurden.

### Psychopharmakologische Behandlung

Die tägliche intraperitoneale Behandlung mit Citalopram (13 mg/kg KG) wurde 7 Tage nach MCAo/Reperfusion begonnen und bis Versuchsende fortgeführt.

### 2. Mirtazapin

Als weitere Substanz wurde das Antidepressivum Mirtazapin getestet, welches ebenfalls intraperitoneal (20 mg/kg KG) beginnend 7 Tage nach MCAo/Reperfusion gegeben wurde. Bei Mirtazapin handelt es sich um ein Antidepressivum, welches präsynaptische alpha2-Rezeptoren blockiert und dadurch die Ausschüttung von Serotonin und Noradrenalin fördert. Da postsynaptisch gleichzeitig 5-HT₂ und 5-HT₃ Rezeptoren blockiert werden, erfolgt die gesteigerte serotonerge Neurotransmission in erster Linie über 5-HT₁ Rezeptoren (noradrenerges und spezifisch serotonerges Antidepressivum).

Die Ergebnisse entsprachen denjenigen, die mit Citalopram erreicht wurden.

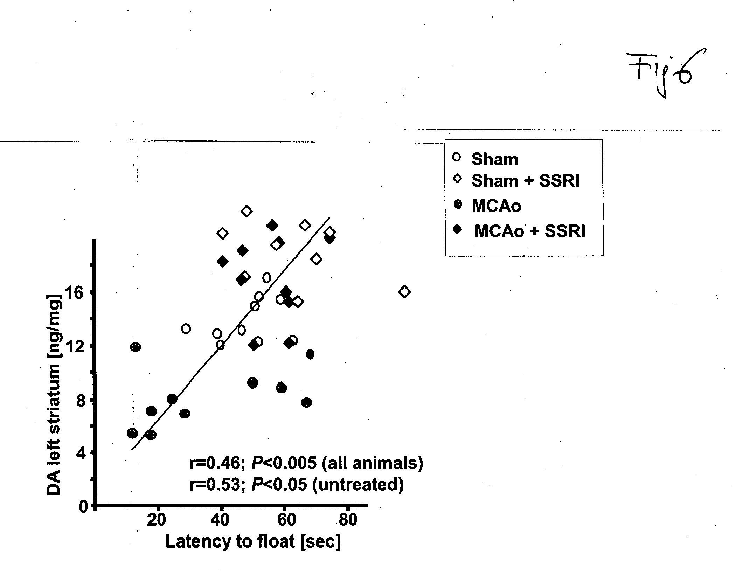

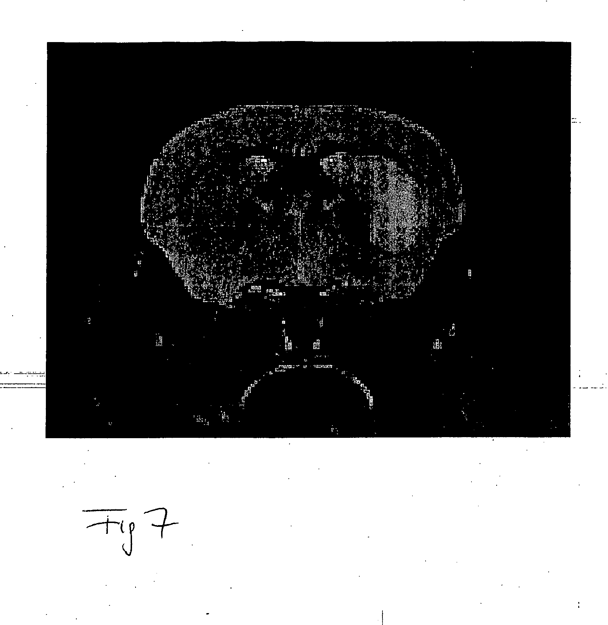

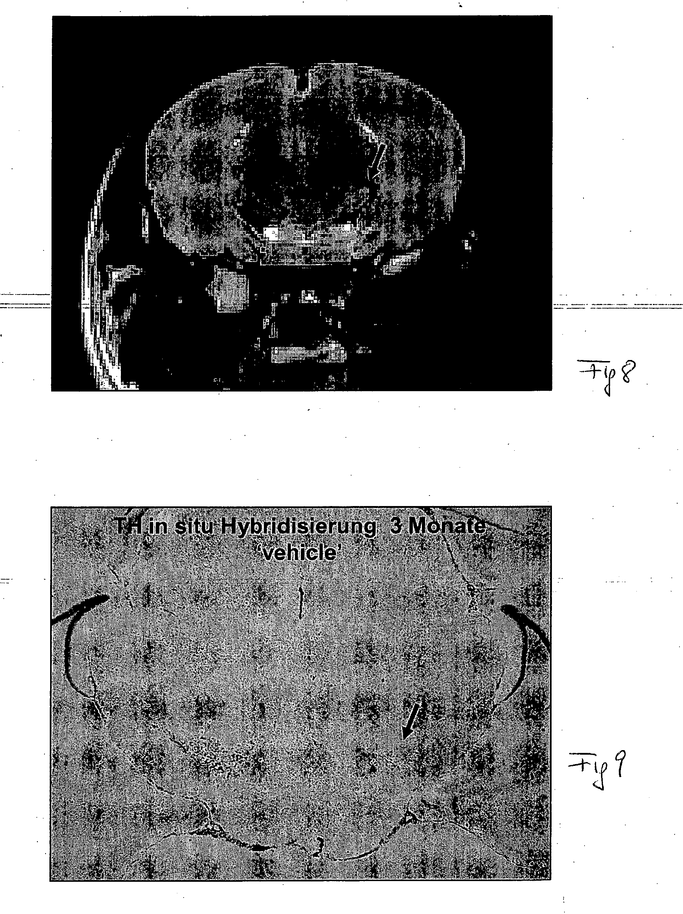

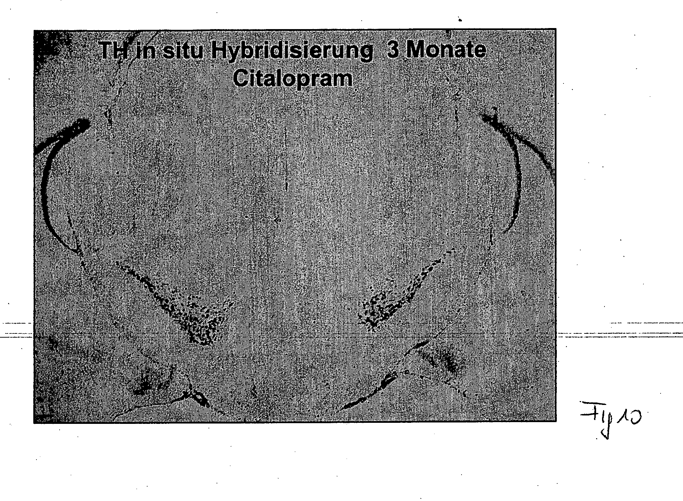

### Figuren 6 bis 10:

- Fig. 6:: Korrelation zwischen Dopaminspiegeln im ipsilateralen (ischämischen) Striatum und Verhalten - protektive Wirkung von Citalopram.
- Fig. 7: Typisches Aussehen des stroke im MR (früher Zeitpunkt) bzw. in der Histologie (MAP2).
- Fig. 8:: Sekundäre Neurodegeneration im MR.
- Fig. 9 und 10:: Tyrosinhydroxylase in situ Hybridisierungen in vehicle - bzw. Citalopram behandeltem Tier

## Patentansprüche

1. Verwendung einer serotonergen Verbindung als Neuroprotektivum zur Behandlung einer fokalen cerebralen Ischämie nach Ablauf der akuten Phase der Ischämie.

2. Verwendung nach Anspuch 1 **dadurch gekennzeichnet, dass** die serotonerge Verbindung ausgewählt ist aus der Gruppe der Verbindungen bestehen aus: SSRI (z.B- Citalopram), Mirtazapin, Amitriptylin, Clomipramin, Desipramin, Doxepin, Imipramin, Maprotilin, Mianserin und Nortriptylen.

3. Verwendung nach Anspruch 1 oder 2, **dadurch gekennzeichnet, dass** die Verbindung nach Ablauf von mindestens etwa 6, vorzugsweise etwa 12 oder etwa 24 Stunden nach Beginn der Ischämie verabreicht wird.

4. Verwendung nach einer der vorherigen Ansprüche, **dadurch gekennzeichnet, dass** die Verbindung zum Ablauf von bis zu 2 bis 60, vorzugsweise 2 bis 30 oder 5 bis 20 Tagen nach Beginn der Ischämie verabreicht wird.

5. Verwendung einer serotonergen Verbindung zur Neuroregeneration bei der Behandlung neuronaler Erkrankungen.

6. Verwendung nach einem der obigen Ansprüche, **dadurch gekennzeichnet, dass** die Verabreichung der serotonergen Verbindung chronisch erfolgt.
